(19) Europäisches Patentamt / European Patent Office / Office européen des brevets

(11) **EP 3 103 492 A1**

(12) **EUROPEAN PATENT APPLICATION**

(43) Date of publication:
**14.12.2016 Bulletin 2016/50**

(51) Int Cl.:
***A61M 5/315*** (2006.01)

(21) Application number: **15171252.8**

(22) Date of filing: **09.06.2015**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>Designated Validation States:<br>**MA** | (71) Applicant: **Sanofi-Aventis Deutschland GmbH 65929 Frankfurt am Main (DE)**<br><br>(72) Inventor: **The designation of the inventor has not yet been filed** |

(54) **DATA COLLECTION APPARATUS FOR ATTACHMENT TO AN INJECTION DEVICE**

(57)  A data collection device comprises a first portion and a second portion, a sensor configured to detect rotation of the second portion relative to first portion and a processor arrangement configured to determine a medicament amount expelled by the injection device based on the detected movement. The first portion may be mounted to a component of the injection device that rotates as medicament is expelled. Alternatively, a rotation transfer means, such as a friction wheel, may be used to engage a rotatable component of the injection device and cause the first portion to rotate. The second portion may include outer and inner portions that are axially movable with respect to one another, to provide an electrical contact therebetween and/or to trigger a timer. The timer may monitor a time elapsed since a previous injection in order to alert the user if a subsequent injection is missed or is attempted early.

FIG. 2

Printed by Jouve, 75001 PARIS (FR)

**Description**

Field of the Invention

The present invention relates to a data collection device for attachment to an injection device and collecting medicament dosage information therefrom.

Background of the Invention

A variety of diseases exists that require regular treatment by injection of a medicament. Such injection can be performed by using injection devices, which are applied either by medical personnel or by patients themselves. As an example, type-1 and type-2 diabetes can be treated by patients themselves by injection of insulin doses, for example once or several times per day. For instance, a pre-filled disposable insulin pen can be used as an injection device. Alternatively, a re-usable pen may be used. A re-usable pen allows replacement of an empty medicament cartridge by a new one. Either pen may come with a set of one-way needles that are replaced before each use. The insulin dose to be injected can then for instance be manually selected at the insulin pen by turning a dosage knob and observing the actual dose from a dose window or display of the insulin pen. The dose is then injected by inserting the needle into a suited skin portion and pressing an injection button of the insulin pen. To be able to monitor insulin injection, for instance to prevent false handling of the insulin pen or to keep track of the doses already applied, it is desirable to measure information related to a condition and/or use of the injection device, such as for instance information on the injected insulin dose.

Summary

According to an aspect, a data collection device comprising includes a first portion configured for attachment to an injection device, a second portion rotatably attached to the first portion, a sensor arrangement configured to detect rotation of the first portion relative to second portion, and a processor arrangement configured to, based on said detected movement, determine a medicament amount expelled by the injection device.

Since the data collection device does not utilise recognition of characters on the injection device to determine a medicament dosage amount, the data collection device may, optionally, be configured so that it does not obscure display of a currently programmed dosage, avoiding the need to provide an additional display of the dosage amount for viewing by the user. In addition, the determination of the medicament dosage amount may be less computationally intensive than optical character recognition techniques.

In one embodiment, the second portion includes an outer portion, having first electrical contacts, and a inner portion, having second electrical contacts, the inner portion being axially movable relative to the outer portion to engage said first electrical contacts with said second electrical contacts when pressure is applied to the outer portion. For instance, engagement of the first and second electrical contacts may activate the data collection device. Alternatively, or additionally, the data collection device may include a timer triggered when said first electrical contacts are engaged and/or disengaged from the second electrical contacts. Such a timer may be used to determine a duration of an injection, if triggered on both engagement and disengagement of the outer and inner portions. Another example use for data from the timer is to determine an elapsed time since the timer was last triggered. For instance, if a user is administering a dosage that is split between two injections, then this could be determined automatically from the timing of trigger events. Yet another example use is to determine if the elapsed time is inconsistent with a threshold condition and, if so, generate an alert. Such an alert may be generated if the elapsed time exceeds a threshold, suggesting a user might have forgotten to administer an injection, or if the elapsed time is much less than a threshold, suggesting a user might be attempting to administer another injection early. The data collection device may also include a display to present the time elapsed since a last injection, based on said trigger events.

The data collection device may be powered by a battery. In some embodiments, a power generation source, such as a solar panel arranged to charge the battery or a piezo-electric generator arranged to power the data collection device when pressure is applied to the outer portion, may be provided.

The data collection device may be configured to switch between a first configuration in which rotation of the first portion relative to the second portion is prevented and a second configuration in which the first portion can be rotated relative to the second portion. For example, the second portion may be axially movable relative to the first portion to switch between said first configuration and said second configuration.

The sensor arrangement comprises one or more of an optical sensor, a magnetic sensor, a capacitive sensor and a mechanical sensor.

The processor arrangement may be configured to obtain time stamp information for the administration of the medicament dosage and to store the determined medicament dosage and said time stamp information. The processor arrangement may be further configured to transmit a log of determined medicament dosages and time stamp information to another device.

The first portion is configured to be attached to a rotatable component of the injection device, such as a rotatable dosage programming component.

Alternatively, the data collection device may include a transfer arrangement, such as a friction wheel arrangement, configured to engage a rotatable component of the injection device, configured to rotate the first portion relative to the second portion when a rotatable component of the injection device rotates. For example, such a transfer arrangement may transfer rotation of a rotatable sleeve, such as a number sleeve, of the injection device.

Another aspect of the invention is a medicament administration apparatus that includes an injection device comprising a rotatable component configured to rotate as a medicament is expelled from the injection device and any of the data collection devices described above.

The injection device may include an injection button arranged to cause expulsion of the medicament from the injection device, in which case the second portion may be arranged to press on the injection button when pressure is applied to the second portion.

In some embodiments, the injection device is a disposable injection device and the data collection device is configured to be releasably attachable to the injection device. In other embodiments, the injection device may be a reusable injection device and the data collection device configured to be permanently attached to the injection device.

The injection device may be an injector pen.

Brief Description of the Drawings

Example embodiments of the invention will now be described with reference to the accompanying figures, of which:

Figure 1 shows an exploded view of an injection device for use with a data collection device according to an embodiment of the invention;

Figure 2 depicts a data collection device according to an embodiment, attached to the injection device of Figure 1;

Figure 3 is a cross-sectional view of the data collection device shown in Figure 2 when attached to the injection device of Figure 1;

Figure 4 depicts locking formations on the data collection device of Figure 2;

Figure 5 is a block diagram of the data collection device of Figure 2;

Figure 6 depicts a system in which data from the data collection device of Figure 2 is transmitted to another device;

Figure 7 is a view of an injection device for use with a data collection device according to another embodiment of the invention;

Figure 8 is a block diagram of a data collection device according to another embodiment, for use with the injection device of Figure 7.

Detailed Description

In the following, embodiments of the present invention will be described with reference to an insulin injection device. The present invention is however not limited to such application and may equally well be deployed with injection devices that eject other medicaments.

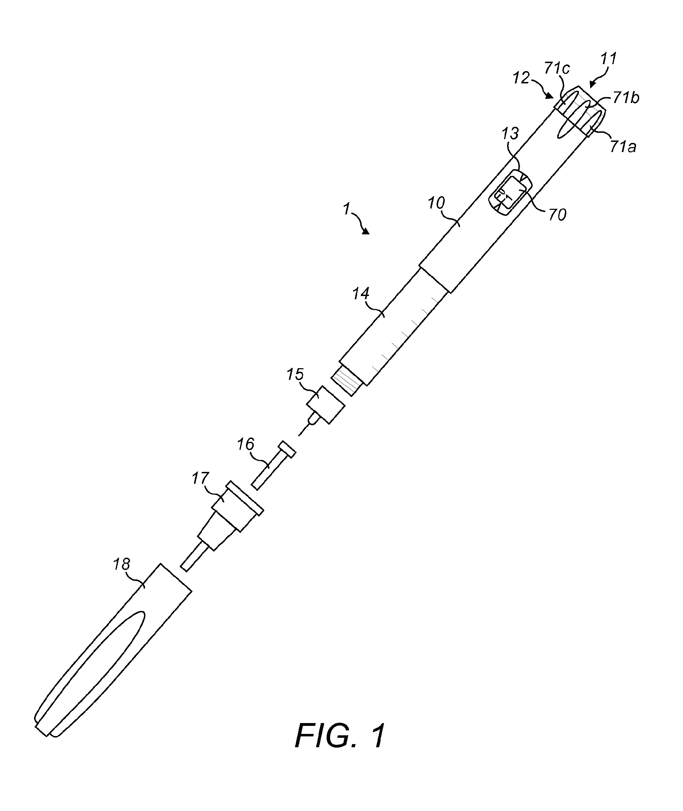

Figure 1 is an exploded view of a medicament delivery device. In this example, the medicament delivery device is an injection device 1, such as Sanofi's SoloSTAR® insulin injection pen.

The injection device 1 of Figure 1 is a pre-filled, disposable injection pen that comprises a housing 10 and contains an insulin container 14, to which a needle 15 can be affixed. The needle is protected by an inner needle cap 16 and either an outer needle cap 17 or an alternative cap 18. An insulin dose to be ejected from injection device 1 can be programmed, or 'dialled in' by turning a dosage knob 12, and a currently programmed dose is then displayed via dosage window 13, for instance in multiples of units. For example, where the injection device 1 is configured to administer human insulin, the dosage may be displayed in so-called International Units (IU), wherein one IU is the biological equivalent of about 45.5 micrograms of pure crystalline insulin (1/22 mg). Other units may be employed in injection devices for delivering analogue insulin or other medicaments. It should be noted that the selected dose may equally well be displayed differently than as shown in the dosage window 13 in Figure 1.

The dosage window 13 may be in the form of an aperture in the housing 10, which permits a user to view a limited portion of a number sleeve 70 that is configured to move when the dosage knob 12 is turned, to provide a visual indication of a currently programmed dose. The dosage knob 12 is rotated on a helical path with respect to the housing 10 when turned during programming.

In this example, the dosage knob 12 includes one or more formations 71 a, 71 b, 71 c that facilitate programming because they improve the grip a user feels when grasping the dosage knob 12. In another example (not shown) the dosage knob does not include formations.

Attaching a data collection device according to the invention device does not require the dosage knob having formations. Having a tight fit and/or using rubber-like material at the contact surface between the data collection device and the injection device would provide an attachment that ensures that on the one hand the connection is stable in the sense that the two devices remain attached to each other, and on the other hand that the two devices can be separated when intended to. The rubber-like material would ensure a proper fit even on a smooth surface, e. g, a dosage knob having a smooth surface such that that rotation of the data collection device causes rotation of the dosage knob and vice versa.

The injection device 1 may be configured so that turning the dosage knob 12 causes a mechanical click sound to provide acoustical feedback to a user. The number sleeve 70 mechanically interacts with a piston in insulin container 14. When needle 15 is stuck into a skin portion of a patient, and then injection button 11 is pushed, the insulin dose displayed in display window 13 will be ejected from injection device 1. When the needle 15 of injection device 1 remains for a certain time in the skin portion after the injection button 11 is pushed, a high percentage of the dose is actually injected into the patient's body. Ejection of the insulin dose may also cause a mechanical click sound, which is however different from the sounds produced when using dosage knob 12.

In this embodiment, during delivery of the insulin dose, the dosage knob 12 is turned to its initial position in an axial movement, that is to say without rotation, while the number sleeve 70 is rotated to return to its initial position, e.g. to display a dose of zero units.

Injection device 1 may be used for several injection processes until either the insulin container 14 is empty or the expiration date of the medicament in the injection device 1 (e.g. 28 days after the first use) is reached.

Furthermore, before using injection device 1 for the first time, it may be necessary to perform a so-called "prime shot" to remove air from insulin container 14 and needle 15, for instance by selecting two units of insulin and pressing injection button 11 while holding injection device 1 with the needle 15 upwards. For simplicity of presentation, in the following, it will be assumed that the ejected amounts substantially correspond to the injected doses, so that, for instance the amount of medicament ejected from the injection device 1 is equal to the dose received by the user. Nevertheless, differences (e.g. losses) between the ejected amounts and the injected doses may need to be taken into account.

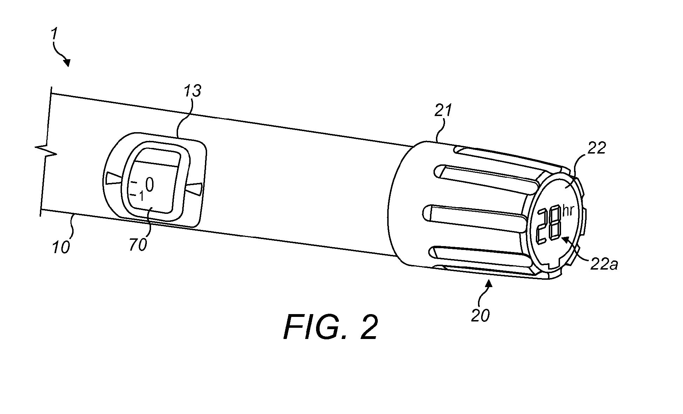

Figure 2 is a perspective view of one end of the injection device 1 when a data collection device 20 according to an example embodiment is attached. The data collection device 20 includes a housing 21 and a end plate 22 with a display 22a.

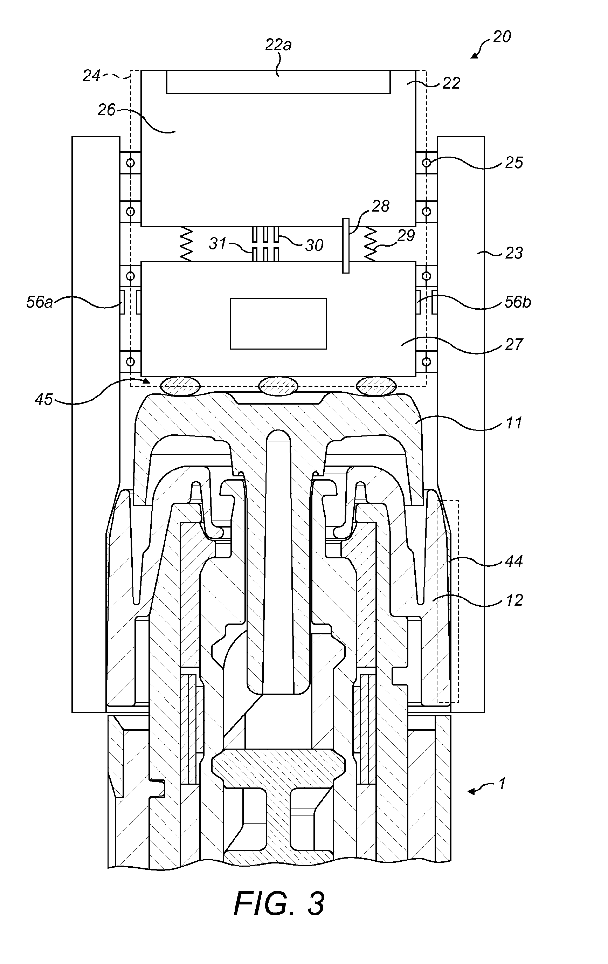

Figure 3 is a cross-sectional view of the data collection device 20 according to an embodiment, when attached to the injection device 1. The data collection device 20 includes a first portion 23 and a second portion 24, where the first portion 23 is capable of rotational movement relative to the second portion 24.

The first portion 23 is a sleeve that is positioned over the dosage knob of the injection device 1. The formations 71 a, 71 b, 71 c on the dosage knob 12 may be used to facilitate attachment of a data collection device 20.

In this particular example, the first portion 23 is a sleeve that is positioned over the dosage knob 12 through formations, not shown, that co-operate with the formations 71 a, 71 b, 71 c on the dosage knob 12 so that, when the dosage knob 12 rotates during programming of the dosage and expulsion of medicament, the first portion 23 also rotates. Alternatively, or additionally, resilient padding, such as a foam rubber pad 44, may be provided within the formations on the first portion 23, to allow for tolerances in the dimensions of the formations on the first portion 23 and the formations 71 a, 71 b, 71 c on the dosage knob 12 and/or to provide an engagement between the first portion 23 and the dosage knob 12 so that rotation of the first portion 23 causes rotation of the dosage knob 12 and vice versa. Further alternatively, the first portion 23 comprises a resilient padding of sufficient thickness to render formations that co-operate with the formations 71 a, 71 b, and 71 c on the dosage knob. The padding is soft enough to conform to the surface of the dosage knob 12. For example, the padding is soft enough to conform to the formations on the surface of the dosage knob 12.

The padding may alternatively or additionally be configured to provide sufficient friction such that an attached data collection device 20 remains in place on the dosage knob 12 and provides an engagement between the first portion 23 and the dosage knob 12 so that rotation of the first portion 23 causes rotation of the dosage knob 12 and vice versa. This example would provide an engagement between the first portion 23 and a dosage knob, wherein the dosage knob does not comprise formations as compares to the example before.

In yet another embodiment, the first portion 23 may be formed with a rigid section surrounding at least part of the second portion 24 and a resilient section that surrounds at least part of the dosage knob 12 when attached to the delivery device 1. The rigid section may provide a firm gripping surface for the user when mounting or removing the data collection device 20 onto or from the injection device 1. Optionally, the rigid section may include formations configured to facilitate operation of the data collection device 20 by improving the grip a user feels when grasping the first portion 23. The resilient section may assist in mounting the first portion 23 over the dosage knob 12 and provide sufficient engagement for transferring rotation between the first portion 23 and dosage knob 12 during operation, such as dose programming and medicament expulsion. The resilient section may be made from a rubber-like material. Initially, when the data collection device 20 is not attached to the injection device 1, the resilient section has an inner diameter that is slightly less than the outer diameter of the dosage knob 12 of the injection device 1. During attachment the data collection device 20 is moved onto the dosage knob 12 thereby widening the resilient section. Thus the data collection device 20 can be easily moved/ slid over the dosage knob 12. The flexible portion provides sufficient elasticity such that the data collection device 20 is releasably fixed to the injection device 1 that it does not fall off or could be removed inadvertently. Moreover, the clamp or press fit-like fastening ensures that when the dosage knob 12 is rotated during programming of the dosage and expulsion of medicament, the first portion 23 also rotates. Optionally, the rigid section may be formed of a different material from the resilient section. A first portion 23 according to the embodiment may, optionally, also include formations configured to co-operate with the formations 71 a, 71 b, 71 c on the dosage knob 12, as described above, and/or resilient padding 44.

To set a medicament dosage amount to be administered, the user may grip and rotate the first portion 23, since this will cause the dosage knob 12 of the injection device 1 to turn and, thereby, program the dosage amount.

Also, in this particular example, the second portion 24 is a body located within the first portion 23, two which it is rotatably attached using bearings 25. The second portion 24 includes an outer portion 26, which includes the endplate 22 and display 22a. The second portion 24 also includes an inner portion 27. When the data collection device 20 is attached to the injection device 1, the inner portion 27 overlies the injection button 11. The outer portion 26 and the inner portion 27 are attached by a fixture 28 that prevents rotation relative to each other. However, in this embodiment, the outer portion 26 can be moved axially relative to the inner portion 27 and one or more resilient members, such as springs 29, may be provided to bias the outer portion 26 away from the inner portion 27.

In this particular arrangement, first electrical contacts 30 are provided on the outer portion 26, while corresponding second electrical contacts 31 are provided on the inner portion 27. When a user presses the endplate 22, the outer portion 26 moves axially towards the inner portion, establishing a connection between the first and second electrical contacts 30, 31. Further pressure on the endplate 22 causes the inner portion 27 to press against, and activate, the injection button 11.

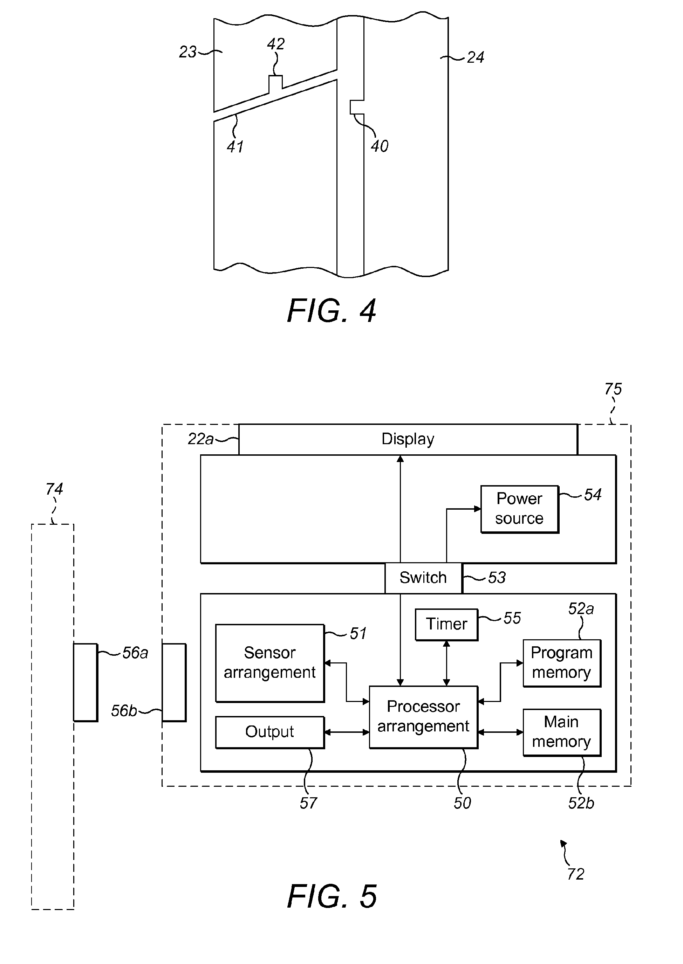

Optionally, the data collection device 20 may be arranged to have a first configuration, in which rotation of the first portion 23 relative to the second portion 24 is prevented, and a second configuration, in which such rotation is unimpeded. For example, as shown in Figure 4, the first portion 23 and the second portion 24 may be provided with lock formations such as a protrusion 40 on one of the first and second portions 23, 24 and a circumferential groove 41 and axial recess 42 on the other of the first and second portions 23, 24. In this particular example, in the first configuration, the protrusion 40 is located in the recess 42 and rotation is prevented. The data collection device 20 may be switched to the second configuration by axial movement of the second portion 24 relative to the first portion 23, so that the protrusion 40 is located within the circumferential groove 41 and rotation is permitted.

While the arrangement shown in Figure 4 included lock formations in the form of a protrusion 40, groove 41 and recess 42, other types of co-operating formations or lock methods may be used to prevent rotation in the first configuration.

Figure 5 is a block diagram of the data collection device 20. In addition to the display 22a, the data collection device 20 includes a processor arrangement 50 including one or more processors, such as a microprocessor, a Digital Signal Processor (DSP), Application Specific Integrated Circuit (ASIC), Field Programmable Gate Array (FPGA) or the like, together with memory units 52a, 52b, including program memory 52a and main memory 52b, which can store software for execution by the processor arrangement 50. The processor is further configured to store data relating to date and /or time information, data relating to information from the sensor output, or combinations thereof. In particular, the memory is configured to store a combination of date and/or time information and dosage information retrieved from the sensor output data. In this way the memory is able to store a medication log that provides a history of information on dose size at a given time. The processor arrangement 50 may be configured to store determined delivered medicament amounts and time stamps for the injections as they are administered. Data can for example be stored in the main memory 52b. Alternatively, data may be stored is a separate data storage section (not shown) of the memory.

The first and second electrical contacts 30, 31 may provide a data connection between the processor arrangement 50 and display 22a when engaged.

A sensor arrangement 51, comprising one or more sensors, is provided for detecting rotational movement between the first portion 23 and the second portion 24.

The resolution of the sensing arrangement 51 is determined by the design of the injection device 1. A suitable angular resolution of the sensing arrangement 51 may be determined by Equation (1):

$$resolution = \frac{360°}{units\_per\_rotation} \qquad (1)$$

For instance, if one full rotation of the dosage knob 12 corresponds to a medicament dosage amount of 24 IU, then a suitable resolution for the sensing arrangement 51 would be not more than 15°.

In this particular example, one or more first magnets 56a are provided around a circumference of the inner surface of the first portion 23 and one or more second magnets 56b are provided around a circumference of the outer surface of the second portion 24, as shown in Figures 3 and 5. The sensor arrangement 26 is a transducer that varies its output due to variations in the magnetic field, based on the Hall effect, as the first portion 23 and first magnets 56a rotate relative to the second portion 24 and second magnets 56b.

Since the first portion 23 rotates with the dosage knob 12 as medicament is expelled from the injection device 1, the angle of rotation measured by the sensing arrangement 51 is proportional to the amount of medicament expelled. The data collection device 20 is thus capable to determine delivered medicament amounts. It is not necessary to determine a zero level or an absolute amount of medicament contained in the injection device 1. In this way the sensor arrangement is less complex than compared to a sensor arrangement that is configured for absolute position detection. Moreover, since it is not necessary to monitor the numbers or tick marks on the number sleeve 70 displayed through the dosage window 13, the data collection device 20 may be designed so that it does not obscure the dosage window 13. The medicament amount delivered is determined by the data collection device 20 independent from the dosage that is programmed into the injection device 1. Determining the delivered medicament amount provides a direct and thus more reliable information about the amount of medicament that is injected compared to data collection devices that determine the amount of medicament that is set, thus being intended to be dispensed.

In other embodiments, different types of sensor may be used. For example, instead of a transducer, the sensor arrangement may include a microelectromechanical (MEMS) device or other magnetic sensor for detecting changes in a magnetic field. Another example of an suitable sensing arrangement would be an optical encoder, including a light source, such as a light emitting diode (LED) and a light detector, such as an optical transducer, that could monitor changes in light reflected from an inner surface of the first portion, where the inner surface first portion has one or regions of varying reflectivity around its circumference, such as tick marks or at least one shaped reflective region.

In other embodiments, the sensing arrangement 51 may be a potentiometer. In yet another example, a capacitive sensing arrangement may be used, where elements provided on the first portion 23 affect the capacitance between two plates in the sensing arrangement. In further examples, mechanical sensors, with mechanical switches and/or tracks, may be used to detect the relative rotation between the first and second portions 23, 25.

While the embodiment described with reference to Figure 5 includes only one type of sensor in the sensor arrangement 51, other embodiments may be devised in which the sensor arrangement 26 includes multiple sensors of one or more types.

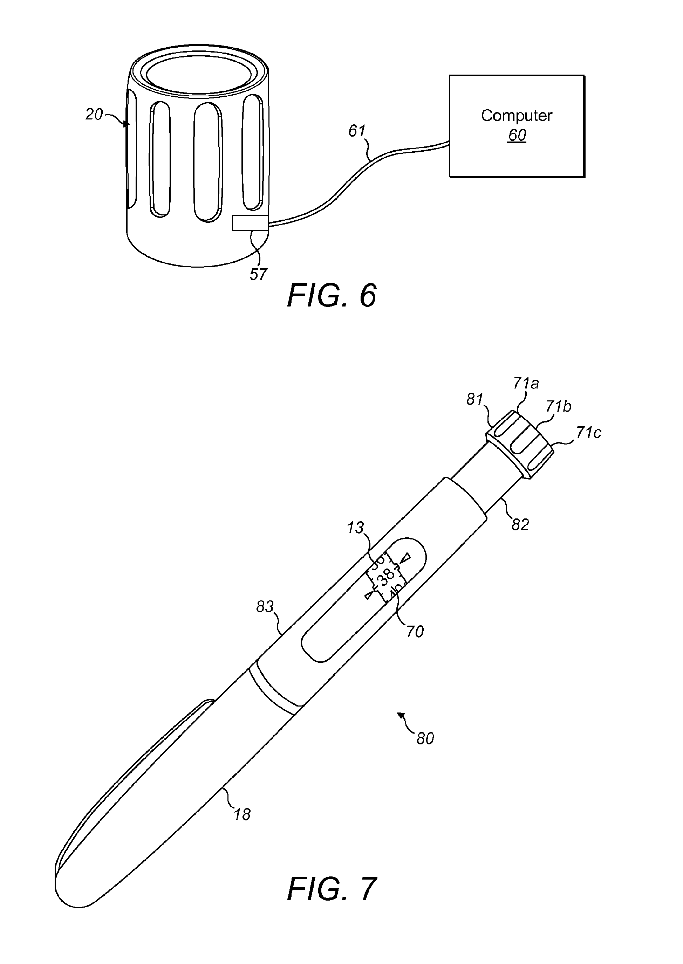

An output 57 is provided, which may be a wireless communications interface for communicating with another device via a wireless network such as wi-fi, Bluetooth®, or NFC, or an interface for a wired communications link, such as a socket for receiving a Universal Series Bus (USB), mini-USB or micro-USB connector. Figure 6 depicts an example of a system in which the data collection device 20 is connected to another device, such as a personal computer 60, via a wired connection 61 for data transfer. For example, the processor arrangement 50 may store determined delivered medicament amounts and time stamps (including date and/or time) for the injections as they are administered by the user and subsequently, transfer that stored data to the computer 60. The computer 60 maintains a treatment log and/or forwards treatment history information to a remote location, for instance, for review by a medical professional.

According to this embodiment, the data collection device 20 is configured to store data such as delivered medicament amounts and time stamps of up to 35 injection events, According to a once-daily injection therapy this would be sufficient to store a treatment history of about one month. Data storage is organized in a first-in first-out manner ensuring that most recent injection events are always present in the memory of the data collection device 20. Once transferred to a computer 60 the injection event history in the data collection device 20 will be deleted. Alternatively, the data remains in the data collection device 20 and the oldest data is deleted automatically once new data is stored. This way the log in the data collection device is built up over time during usage and will always comprise the most recent injection events. Alternatively, other configuration could comprise a storage capacity of 70 (twice daily), 100 (three months) or any other suitable number of injection events depending on the therapy requirements and/or the preferences of the user.

In another embodiment, the output 57 may be configured to transmit information using a wireless communications link and/or the processor arrangement 23 may be configured to transmit such information to the computer 40 periodically.

A power switch 58 is provided, together with a power source 59. In some embodiments, the power switch 58 may be provided by the first and second electrical contacts 30, 31, where the power switch 58 responds to pressure applied to the display 22a by powering the data collection device 20 on or off, so that power may conserved when the injection device 1 is not being used. In such an arrangement, the data collection device 20 is powered on again, the processor arrangement 50 may control the display 22a to show the determined medicament dose information 22a, to aid the memory of the user, and/or an elapsed time since the determined medicament dose was delivered. For example, the processor arrangement 23 may cause the display 22 to switch periodically between displaying the most recent determined medicament dosage information and the elapsed time.

The power source 59 may be a battery. In some embodiments, the endplate 22 may include a solar panel to charge such a battery.

In another embodiment, the power source 59 may be a piezo-electric generator, which generates power when the endplate 22 is pressed by the user, potentially avoiding the need for a battery.

A timer 55 is also provided. In addition to, or instead of, switching the data collection device 20 on and off, the first and second electrical contacts 30, 31 may be arranged to trigger the timer 55 when engaged and/or disengaged. For example, if the timer 55 is triggered on both engagement or disengagement of the first and second electrical contacts 30, 31, then the processor arrangement 50 may use the output from the timer to determine a length of time during which the injection button 11 was pressed, for example to determine the duration of an injection.

Alternatively, or additionally, the processor arrangement 50 may use the timer 55 to monitor a length of time that has elapsed since an injection was completed, as indicated by a time of disengagement of the first and second electrical contacts 30, 31. Optionally, the elapsed time may be shown on the display 22a, as depicted in Figure 2. Also optionally, when the first and second contacts 30, 31 are next engaged, the processor arrangement 50 may compare the elapsed time with a predetermined threshold, to determine whether a user may be attempting to administer another injection too soon after a previous injection and, if so, generate an alert such as an audible signal and/or a warning message on the display 22a. On the other hand, if the elapsed time is very short, it may indicate that the user is administering a medicament amount as a "split dose", and the processor arrangement 50 may store information indicating that a dosage was delivered in that manner. In such a scenario the elapsed time is compared with a predetermined threshold in the range of a few seconds, e.g. 10 seconds up to a few minutes, e.g. 5 minutes. According to an example the predetermined threshold is set to 2 minutes. If the time elapsed since the last injection is two minutes or less, the processor arrangement 50 stores information indicating that the dosage was delivered as a "split dose".

Another optional purpose for monitoring the elapsed time by the processor arrangement 50 is to determine when the elapsed time has passed a predetermined threshold, suggesting that the user might have forgotten to administer another injection and, if so, generate an alert.

The data collection device 20 is used where the injection device 1 includes a component that can rotate as medicament is dispensed. However, in some injection devices, the dosage knob does not rotate during dose delivery. However, there might be another rotatable component close to the dose knob to which the first portion 23 can be attached directly or indirectly. Figure 7 depicts an example of such an injection device 80. In Figure 7, components similar to those of the injection device 1 shown in Figure 1 are indicated using the same reference labels. Also, the injection device 80 is shown with its cap 18 in place, covering the needle and medicament chamber.

In this particular example of an injection device 80, the injection button and dosage knob are provided by a single component, knob 81. A medicament dosage can be programmed into the injection device 72 by turning the knob 81, which rotates a sleeve 82 which extends outwards from a housing 83 of the injection device 70 as the programmed dosage increases. As described above in relation to Figure 1, turning the knob 81 also turns a number sleeve 70, so that a dosage amount is displayed in the dosage window 13.

When the knob 81 is pressed to administer an injection or prime shot, the knob 81 is partially decoupled from the sleeve 82, so they can rotate independently of one another. As medicament is expelled from the injection device 80, the sleeve 82 rotates and moves into the housing 83. Meanwhile, the knob 81 moves towards the housing but does not rotate.

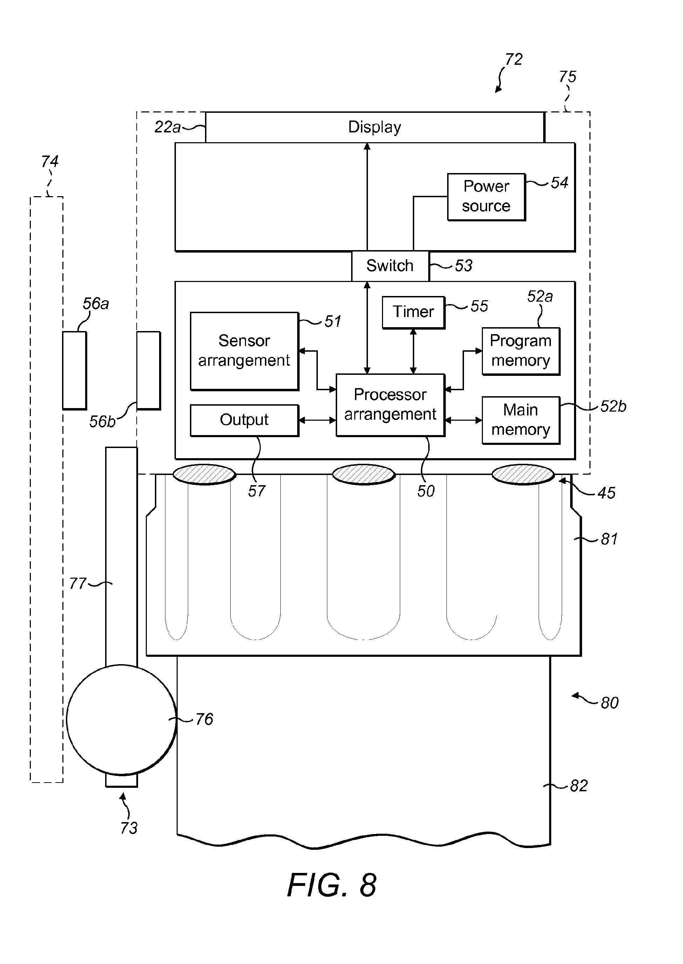

Figure 8 is a block diagram of a data collection device 72 according to a further embodiment, which is capable of being used with the injection device 80 of Figure 7 that does not have a rotatable component to which the first portion can be directly attached. Components of the data collection device 72 that correspond to those of the data collection device 20 of Figure 2 are shown with the same reference labels.

The data collection device 72 is provided with a transfer arrangement 73. The transfer arrangement 73 is configured so that, when the data collection device 72 is attached to the injection device, it engages a component of the injection device that rotates as medicament is expelled. The transfer arrangement 73 responds to rotation of the rotatable component by causing the first portion 74 to rotate relative to the second portion 75.

In this particular example, the transfer arrangement 73 is mounted on the second portion 75 includes a friction wheel 76. In this particular example, the friction wheel 76 has a substantially spherical, or ball, shape, so that the friction wheel 76 can pass over the knob 81 when the data collection device 72 is mounted to the injection device 80 and to allow for axial movement between the first portion 74 and second portion 75, for example when using a locking mechanism similar to that described with reference to the data collection device 20 and Figure 4. The friction wheel 76 may be attached to the second portion 75 by a resilient arm 77, to facilitate movement of the friction wheel 76 over the knob 81 when mounting or removing the data collection device 72.

While Figure 8 depicts an embodiment in which the transfer arrangement 73 includes one friction wheel 76, other embodiments may include one or more additional friction wheels 76, such as two or three friction wheels. The provision of such additional friction wheels 76 may improve the reliability of transfer of rotation between the sleeve 82 and first portion 74.

As shown in Figure 8, the knob 81 of the injection device 80 is in contact with the second portion 75. One or more resilient pads 45 may be provided between the second portion 75 and knob 81 to improve engagement therebetween. One or more of formations, not shown, configured to co-operate with formations 71 a, 71 b, 71 c on the knob 81 of the injection device 80, resilient pads 84 and resilient portions in the second portion 75 may be provided to enhance engagement between the second portion 75 and the knob 81, so that rotation of the second portion 75 causes rotation of the knob 81 and vice versa.

However, there first portion 74 does not contact, or engage directly with, the knob 81. Therefore, the first portion 74 and second portion 75 may be locked together, as described above in relation to Figure 4, so that the injection device 80 can be programmed by turning the first portion 74, to rotate the second portion 75 and, therefore, the knob 81. When the dosage amount has been programmed, the user may press the second portion 75, which in turn depresses the knob 81, to dispense the programmed amount of medicament from the injection device 80. The axial movement of the second portion 75 relative to the first portion 74, releases the locking mechanism that prevents rotation of the first portion 74 relative to the second portion 75.

As the medicament is expelled, the knob 81 moves towards the housing 83 but does not rotate. The sleeve 82 rotates while it moves back into the housing 83. The rotation of the sleeve 82 is transferred to the first portion 74 through the transfer arrangement 73. Therefore, the first portion 74 is caused to rotate relative to the second portion 75. The sensor arrangement 51 then detects the angle of rotation from which the medicament dosage amount can be determined, as described above in relation to the data collection device 20 of Figure 2.

The specific embodiments described in detail above are intended merely as examples of how the present invention may be implemented. Many variations in the configuration of the data collection device 20, 72 and/or the injection device 1, 80 may be conceived.

In particular, while the embodiments above have been described in relation to collecting data from an insulin injector pen, it is noted that embodiments of the invention may be used for other purposes, such as monitoring of injections of other medicaments.

**Claims**

1. A data collection device comprising:

   a first portion configured for attachment to an injection device;
   a second portion rotatably attached to the first portion;
   a sensor arrangement configured to detect rotation of the first portion relative to the second portion; and
   a processor arrangement configured to, based on said detected movement, determine a medicament amount expelled by the injection device.

2. A data collection device according to claim 1, wherein:

   the second portion comprises an outer portion having first electrical contacts;
   the second portion comprises a inner portion having second electrical contacts; and
   the inner portion is axially movable relative to the outer portion to engage said first electrical contacts with said second electrical contacts when pressure is applied to the outer portion.

3. A data collection device according to claim 2, comprising a piezo-electric generator arranged to power the data collection device when pressure is applied to the outer portion.

4. A data collection device according to claim 2 or 3, comprising a timer triggered when said first electrical contacts are engaged and/or disengaged from the second electrical contacts.

5. A data collection device according to claim 4, configured to determine an elapsed time since the timer was last triggered and to generate an alert if the elapsed time is inconsistent with a threshold condition.

6. A data collection device according to claim 4 or 5, wherein the processor arrangement is configured to determine a time stamp for the administration of the medicament dosage using said timer and to store the determined medicament dosage and said time stamp.

7. A data collection device according to claim 6, wherein the processor arrangement is configured to transmit a log of determined medicament dosages and time stamp information to another device.

8. A data collection device according to any of the preceding claims, configured to switch between a first configuration in which rotation of the first portion relative to the second portion is prevented and a second configuration in which the first portion can be rotated relative to the second portion.

9. A data collection device according to any of the preceding claims, wherein the sensor arrangement comprises one or more of an optical sensor, a magnetic sensor, a capacitive sensor and a mechanical sensor.

10. A data collection device according to any of the preceding claims, wherein the first portion is configured to be attached to a rotatable component of the injection device.

11. A data collection device according to any of claims 1 to 10, comprising:

a transfer arrangement arranged to engage a rotatable component of the injection device;
wherein:

the first portion is configured to be attached to a rotatable component of the injection device; and
the transfer arrangement is configured to rotate the first portion relative to the second portion when said rotatable component rotates.

12. A data collection device according to claim 11, wherein said transfer arrangement comprises a friction wheel.

13. A medicament administration apparatus comprising:

an injection device comprising a rotatable component configured to rotate as a medicament is expelled from the injection device; and
a data collection device according to any of claims 1 to 12.

14. A medicament administration apparatus according to claim 13, wherein:

the injection device comprises an injection button arranged to cause expulsion of the medicament from the injection device; and
the second portion is arranged to press on the injection button when pressure is applied to the second portion.

15. A medicament administration apparatus comprising:

an injection device comprising a rotatable component configured to rotate as a medicament is expelled from the injection device; and
a data collection device according to claim 12;
wherein the transfer arrangement is arranged to engage a rotatable sleeve of the injection device.

FIG. 1

FIG. 2

**FIG. 3**

**FIG. 4**

**FIG. 5**

FIG. 6

FIG. 7

**FIG. 8**

Europäisches
Patentamt

European
Patent Office

Office européen
des brevets

# EUROPEAN SEARCH REPORT

Application Number

EP 15 17 1252

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/098927 A1 (LIFESCAN INC [US]; KRULEVITCH PETER [US]; WILK ROBERT [US]; KRAFT ULRI) 2 September 2010 (2010-09-02) | 1 | INV. A61M5/315 |
| Y | * paragraph [0057] - paragraph [0060]; figures 6A-8A * | 2-15 | |
| X | EP 2 692 378 A1 (SANOFI AVENTIS DEUTSCHLAND [DE]) 5 February 2014 (2014-02-05) | 1 | |
| Y | * figures * | 2-15 | |
| X | WO 2014/037331 A1 (SANOFI AVENTIS DEUTSCHLAND [DE]) 13 March 2014 (2014-03-13) | 1 | |
| Y | * figure 11 * | 2-15 | |
| Y | DE 299 04 864 U1 (BRAUN MELSUNGEN AG [DE]) 3 August 2000 (2000-08-03) * figures 6,7 * | 1-15 | |
| Y | EP 2 762 184 A1 (SANOFI AVENTIS DEUTSCHLAND [DE]) 6 August 2014 (2014-08-06) * figures 3,4 * | 1-15 | TECHNICAL FIELDS SEARCHED (IPC) A61M |
| X | WO 2014/064691 A (INSULINE MEDICAL LTD.) 1 May 2014 (2014-05-01) | 1 | |
| Y | * abstract; figure 1 * | 2-15 | |

The present search report has been drawn up for all claims

| Place of search | Date of completion of the search | Examiner |
|---|---|---|
| Munich | 1 December 2015 | Ehrsam, Fernand |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................
& : member of the same patent family, corresponding
document

EPO FORM 1503 03.82 (P04C01)

**EP 3 103 492 A1**

## ANNEX TO THE EUROPEAN SEARCH REPORT
## ON EUROPEAN PATENT APPLICATION NO.

EP 15 17 1252

This annex lists the patent family members relating to the patent documents cited in the above-mentioned European search report.
The members are as contained in the European Patent Office EDP file on
The European Patent Office is in no way liable for these particulars which are merely given for the purpose of information.

01-12-2015

| Patent document cited in search report | | Publication date | Patent family member(s) | | Publication date |
|---|---|---|---|---|---|
| WO 2010098927 | A1 | 02-09-2010 | CA | 2753069 A1 | 02-09-2010 |
| | | | CA | 2753138 A1 | 02-09-2010 |
| | | | CA | 2753139 A1 | 02-09-2010 |
| | | | CA | 2753140 A1 | 02-09-2010 |
| | | | CN | 102413759 A | 11-04-2012 |
| | | | CN | 102413852 A | 11-04-2012 |
| | | | CN | 102413855 A | 11-04-2012 |
| | | | CN | 102413856 A | 11-04-2012 |
| | | | CN | 103520806 A | 22-01-2014 |
| | | | CN | 104888316 A | 09-09-2015 |
| | | | EP | 2400883 A1 | 04-01-2012 |
| | | | EP | 2401006 A1 | 04-01-2012 |
| | | | EP | 2401011 A1 | 04-01-2012 |
| | | | EP | 2401012 A1 | 04-01-2012 |
| | | | EP | 2767297 A2 | 20-08-2014 |
| | | | EP | 2926846 A1 | 07-10-2015 |
| | | | ES | 2478065 T3 | 18-07-2014 |
| | | | HK | 1164764 A1 | 06-03-2015 |
| | | | HK | 1201223 A1 | 28-08-2015 |
| | | | JP | 5684738 B2 | 18-03-2015 |
| | | | JP | 5711155 B2 | 30-04-2015 |
| | | | JP | 5711156 B2 | 30-04-2015 |
| | | | JP | 5711157 B2 | 30-04-2015 |
| | | | JP | 2012519025 A | 23-08-2012 |
| | | | JP | 2012519026 A | 23-08-2012 |
| | | | JP | 2012519027 A | 23-08-2012 |
| | | | JP | 2012519028 A | 23-08-2012 |
| | | | US | 2011313349 A1 | 22-12-2011 |
| | | | US | 2011313350 A1 | 22-12-2011 |
| | | | US | 2011313395 A1 | 22-12-2011 |
| | | | US | 2012004637 A1 | 05-01-2012 |
| | | | WO | 2010098927 A1 | 02-09-2010 |
| | | | WO | 2010098928 A1 | 02-09-2010 |
| | | | WO | 2010098929 A1 | 02-09-2010 |
| | | | WO | 2010098931 A1 | 02-09-2010 |
| EP 2692378 | A1 | 05-02-2014 | NONE | | |
| WO 2014037331 | A1 | 13-03-2014 | NONE | | |
| DE 29904864 | U1 | 03-08-2000 | AT | 346624 T | 15-12-2006 |
| | | | DE | 29904864 U1 | 03-08-2000 |
| | | | EP | 1043037 A2 | 11-10-2000 |
| | | | ES | 2273628 T3 | 16-05-2007 |
| | | | US | 6482185 B1 | 19-11-2002 |

EPO FORM P0459

For more details about this annex : see Official Journal of the European Patent Office, No. 12/82

EP 3 103 492 A1

## ANNEX TO THE EUROPEAN SEARCH REPORT
## ON EUROPEAN PATENT APPLICATION NO.

EP 15 17 1252

This annex lists the patent family members relating to the patent documents cited in the above-mentioned European search report.
The members are as contained in the European Patent Office EDP file on
The European Patent Office is in no way liable for these particulars which are merely given for the purpose of information.

01-12-2015

| Patent document cited in search report | | Publication date | Patent family member(s) | | Publication date |
|---|---|---|---|---|---|
| EP 2762184 | A1 | 06-08-2014 | NONE | | |
| WO 2014064691 | A | 01-05-2014 | EP | 2911717 A2 | 02-09-2015 |
| | | | US | 2015290396 A1 | 15-10-2015 |
| | | | WO | 2014064691 A2 | 01-05-2014 |

EPO FORM P0459

For more details about this annex : see Official Journal of the European Patent Office, No. 12/82